# EUROPEAN PATENT SPECIFICATION

(11) **EP 0 328 179 B1**
(45) Date of publication and mention of the grant of the patent: **17.02.1993**
(21) Application number: 89200174.4
(22) Date of filing: 27.01.1989
(51) Int. Cl.: C07C 231/10, C07C 233/34

(54) **Method of preparing low color fatty amides**
Verfahren zur Herstellung von wenig gefärbten Fettsäureamiden
Procédé pour la préparation d'amides d'acides gras légèrement colorés

(30) Priority: 09.02.1988 US 153861
(43) Date of publication of application: 16.08.1989
(73) Proprietor: AKZO N.V., NL-6824 BM Arnhem (NL)
(72) Inventor: Bailey, Bruce Raymond, Naperville Illinois 60565 (US); Richmond, James Matthew, Naperville Illinois 60565 (US)
(74) Representative: Schalkwijk, Pieter Cornelis, c.s.

(56) References cited:
- EP-A- 0 164 205
- WO-A-79/00628
- FR-A- 2 010 727
- US-A- 2 828 320
- CHEMICAL ABSTRACTS, vol. 84, 17th May 1976, page 55, abstract no. 136749t, Columbus, Ohio, US; & JP-A-75 151 992

## Description

This invention relates to the manufacture of fatty acid amides from fatty acids and amines, particularly alkyl polyamines. In particular, it relates to such methods which yield fatty acid amides with relatively low color.

Fatty amides made from a fatty acid and an amine (particularly an alkylene polyamine) have utility as fabric softeners. Fatty materials in general, fatty amides being no exception, are prone to developing undesirable color upon chemical reaction, heating and storage. Consumer uses for fatty acid amides require low color; therefore, high color fatty amides are not saleable in such applications.

One approach to decoloring fatty materials is to recrystallize the product; however, this causes product loss and is otherwise expensive. Another approach is to distill the colored product, but this again is expensive and can result in additional color formation if heat is used in the distillation. Yet another approach is the use of liquified hydrocarbon gases, such as propane, to extract colored components. This approach, however, is also expensive and poses an additional safety hazard because of the flammable or explosive nature of such gases. One problem unaddressed by these approaches is that, even after decolorization, the fatty materials will tend to become colored during storage.

US2828320 teaches color stabilization of previously decolored fatty acids with a blend of hypophosphorous acid and one of di-tertiarybutyl para cresol (BHT), butylated hydroxy anisol, beta naphthol, propyl gallate or hydroquinone. The patent does not, however, address the the problem of color formation during reaction or the possible reaction of the stabilizer materials with the intended reactants.

Japanese publication 35528/1970 specifically addresses the production of fatty acid bisamides, and uses a mixture of (1) a phosphorous acid, a hypophosphorous acid, or an alkali or alkaline earth salt thereof, and (2) an alkali borohydrate. This process, however, does not stabilize the color as much as would be desired.

The present invention overcomes these disadvantages by providing a method of preparing a low color fatty acid amide from a fatty acid and an amine, charaterized in that the fatty acid and amine are reacted in the presence of hypophosphorous acid and a hindered phenol. More preferably, the components are also reacted in the substantial absence of oxygen.

A first component of the invention is a fatty acid, preferably a C₄ to C₂₂ carboxylic acid. Such carboxylic acids are commonly referred to as fatty acids, and are typically derived from natural sources such as pressed plant oils and slaughter house scrap. Because of their natural origin, such acids will usually be a mixture of similar acids rather than a single species. These fatty acids may be either saturated or unsaturated. If in its "natural" form such an acid is unsaturated, it may be partially or completely hydrogenated to reduce or eliminate unsaturation. Of the aforementioned C₄ to C₂₂ fatty acids, the C₆ to C₂₂ fatty acids are desired, and the C₈ to C₂₂ fatty acids are more desired. Particularly preferred are the C₁₂ to C₁₈ fatty acids such as tallow acid and hydrogenated tallow acid.

For the purposes of this invention, fatty acid shall also be meant to include esters of the fatty acids. In particular, triglycerides of fatty acids are convenient because of their ready availability from animal sources.

A second component of the invention is an amine. Suitable amines include simple monoamines such as ethylamine, fatty monoamines such as tallow amine, and alkylene polyamines. In a preferred embodiment of the invention, the amine is an alkylene polyamine of the general formula (I)${\text{NH₂(CH₂)}}_{\text{m}} {\text{[NH(CH₂)}}_{\text{n}} {\text{]}}_{\text{p}} \text{NH₂}$
wherein m and n are independently from 2 to 8, and p is from 0 to 3. Although it is not required, it is preferred that the polyamine be symmetrical, that is, the values of m and n being the same. Further, it is preferred that p = 0 or 1, and most preferred that p = 1. Diethylenetriamine (m, n = 2 and p = 1) is especially preferred, with each higher analog (m, n = 3 to 8) being succeedingly less preferred because of the lower reactivity in the method of the invention.

A third component of the invention is hypophosphorous acid (H₃PO₂), including salts thereof. If salts are used, it is preferred that they be alkali or alkaline earth salts. Of the salts, the sodium and potassium salts are preferred; however, the acid form is most preferred. Hypophosphorous acid is typically sold in 30-50% aqueous solutions and may be used as such.

A fourth component of the invention is a hindered phenol compound. By "hindered phenol compound" is meant an aromatic compound having a hydroxyl group attached directly to the aromatic ring, and at least one moiety ortho the hydroxyl which causes steric hinderence. To be considered a hindered phenol, a compound must function as an antioxidant by abstraction of free radicals.

As an example of a hindered phenol suitable for use in the method of the present invention may be mentioned compounds of the general formula(II) 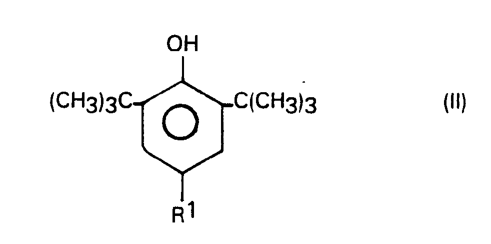
wherein R₁ is hydrogen or an organic moiety such as a lower alkyl e.g., methyl, ethyl, etc.) or a polymeric material. Preferred of these, when R1 is a lower alkyl, is butylated hydroxytoluene (BHT) (2,6-di-tert-butyl-para-cresol). Also preferred are those wherein R¹ is of a relatively high molecular weight, because higher molecular weight hindered phenols have generally been found to be more effective in the invention. As especially preferred example of these may be mentioned generally the higher molecular weight hindered phenols sold under the trademark "IRGANOX" (Ciba-Geigy Corp.), such as Irganox® 1076.

Other examples of suitable hindered phenols include butylated hydroxyanisole (BHA) (a mixture of 2- and 3-tert-butyl-4-methoxy phenol), and other higher molecular weight hindered phenols also sold under the trademark "IRGANOX" (Ciba-Geigy Corp.), such as Irganox® 1010.

The fatty acid is reacted with the amine using, except for the addition of the hypophosphorous acid and the hindered phenol, conventional techniques and conditions. The fatty acid and amine are utilized generally in an equimolar ratio with a slight (e.g., about 1% by weight) excess of amine preferred to allow for loss in the reaction apparatus.

In a typical procedure according to the present invention, the fatty acid, hypophosphorous acid and hindered phenol are charged to a reactor, which is purged with nitrogen to remove essentially all of the oxygen. The amine is then added, the pressure reduced to 3 to 50 kPa (absolute), more preferably 3 to 7 kPa, and the reaction mixture heated to 160°C to 250°C, more preferably 180°C to 225°C, for 0,5 to 50 hours, more preferably 1 to 20 hours, still more preferably 1 to 4 hours. The reduced pressure is greatly preferred for removal of reaction water (glycerine if triglycerides are utilized) and to reduce the reaction time. Additionally, the elevated temperature is preferred for complete reaction. The use of a solvent is not necessary, but may be used to assist with removal of the product from the reactor.

The aforementioned process of the invention is very sensitive to minor variations in the reaction procedure. One of the most critical factors is the exclusion of oxygen from the system, as allowing even minor amounts of oxygen to enter the reaction vessel may cause significant color. Thus it is preferred to take suitable measures to exclude oxygen, such as maintaining a nitrogen flow of about 0,005 m3/hr/kg of reactant, which also assists in removing reaction water.

In the preferred embodiment wherein the amine is diethylenetriamine, the product is a dialkylamidoethylimidazoline of the general formula (III) 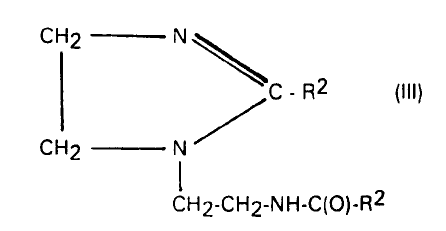
wherein each R² represents the residue of the particular fatty acid used in the reaction.

The hypophosphorous acid and the hindered phenol compound are each present in a "synergistic" mixture. By "synergisitc" mixture is it meant that each is present, relative to the other, in an amount such that the mixture is more effective in reducing color in the product than an equivalent weight of either component alone. Further, the hypophosphorous acid and the hindered phenol compound are used together in a "color stabilizing" amount. By "color stabilizing" amount is meant an amount of the mixture, in relation to the reactants and product, which is sufficient to render the product less colored than if the hypophosphorous acid and the hindered phenol compound were not present.

In light of the above, the hypophosphorous acid is preferably present in amounts from 0,005 to 0,5 weight percent, more preferably from 0,01 to 0,25 weight percent, and still more preferably from 0,05 to 0,1 weight percent, based upon the weight of the total reaction charge (fatty acid plus amine). Further, the hindered phenol is preferably present in amounts from 0,001 to 5,0 weight percent, more preferably from 0,01 to 3,0 weight percent, and still more preferably from 0,1 to 1,0 weight percent, based upon the weight of the total reaction charge (fatty acid plus amine).

The color of the amides produced by the process of the present invention is very low, and remains relatively low during storage. The storage stability can be further increased by a post-reaction addition of the hindered phenol, but the post reaction addition is not so important when the preferred high molecular weight hindered phenols are used in the initial reaction charge. If the hindered phenol is so added as a post treatment, it should be utilized in a "post treatment color stabilizing" amount. By "post treatment color stabilizing" amount is meant an amount of the hindered phenol which, when added subsequent to the reaction, will render the amide more color stable than if it were not added. As a post treatment, the hindered phenol is preferably added in an amount from 0,005 to 0,5 weight percent, more preferably from 0,01 to 0,25 weight percent, still more preferably from 0,05 to 0,1 weight percent, based upon the weight of the total reaction charge (fatty acid plus amine).

The invention will be further illustrated by the following example, in which all parts and percentages are by weight unless otherwise specified.

### Theoretical Example 1

A reaction vessel is fitted with a vacuum source and a nitrogen source. The vessel is charged with 1 equivalent tallow acid, 0,1% BHT, and 0,1% of a 50% aqueous solution of hypophosphorous acid (both percents are based upon the fatty acid plus polyamine). The vessel is flushed with nitrogen to remove all oxygen and 1 equivalent diethylene triamine is added. The vessel is heated to 180°C to 225°C under vacuum with a nitrogen flow. After 2 hours, the resulting product should show a high degree of ring closure and a very low Gardner color value. If desired, prior to storage 0,1% BHT may additionally be added.

## Claims

1. A method of preparing a low color fatty acid amide from a C₄ to C₂₂ fatty acid and an amine, characterized in that the fatty acid and amine are reacted in the presence of
(a) 0,005 to 0,5 weight percent of hypophosphorous acid, and
(b) 0,001 to 5,0 weight percent of a hindered phenol compound, wherein weight percent is based upon the weight of the fatty acid and amine.

2. The method according to claim 1, characterized in that the fatty acid and amine are reacted, in the presence of hypophosphorous acid and the hindered phenol compound, in the substantial absence of oxygen.

3. The method according to claims 1 or 2, characterized in that the amine comprises an alkylene polyamine of the general formula${\text{NH₂(CH₂)}}_{\text{m}} {\text{[NH(CH₂)}}_{\text{n}} {\text{]}}_{\text{p}} \text{NH₂}$ wherein m and n are independently from 2 to 8, and p is from 0 to 3.

## Patentansprüche

1. Verfahren zur Herstellung eines wenig gefärbten Fettsäureamids aus einer C₄ bis C₂₂ Fettsäure und einem Amin, dadurch gekennzeichnet, dass die Fettsäure und das Amin umgesetzt werden in Gegenwart von:
(a) 0,005 bis 0,5 Gew.% Phosphinsäure und
(b) 0,001 bis 5,0 Gew.% einer behinderten Phenolverbindung worin die Gewichtsprozente auf das Gewicht der Fettsäure und des Amins bezogen sind.

2. Verfahren nach Anspruch 1, dadurch gekennzeichnet, dass die Fettsäure und Amin in Gegenwart von Phosphinsäure und der behinderten Phenolverbindung in praktischer Abwesenheit von Sauerstoff umgesetzt werden.

3. Verfahren nach den Ansprüchen 1 oder 2, dadurch gekennzeichnet, dass das Amin ein Alkylenpolyamin der allgemeinen Formel${\text{NH₂(CH₂)}}_{\text{m}} {\text{[NH(CH₂)}}_{\text{n}} {\text{]}}_{\text{p}} \text{NH₂}$ enthält, in der m und n unabhängig voneinander 2 bis 8 sind und p Null bis 3 ist.

## Revendications

1. Un procédé de préparation d'amide d'acide gras présentant une faible coloration à partir d'acide gras en C₄ à C₂₂ et d'une amine, caractérisé en ce que l'acide gras et l'amine réagissent en présence de:
(a) 0,005 à 0,5% en poids d'acide hypophosphoreux; et
(b) 0,001 à 5,0% en poids d'un phénol encombré, dans lequel les pourcentages en poids sont calculés sur la base, en poids, d'acide gras plus amine.

2. Le procédé selon la revendication 1, caractérisé en ce que l'acide gras et l'amine réagissent en présence d'acide hypophosphoreux et du phénol encombré, en l'absence substantielle d'oxygène.

3. Le procédé selon la revendication 1 ou 2, caractérisé en ce que l'amine comprend une alkylène polyamine de formule générale:${\text{NH₂(CH₂)}}_{\text{m}} {\text{[NH(CH₂)}}_{\text{n}} {\text{]}}_{\text{p}} \text{NH₂}$ dans laquelle:
m et n sont indépendamment compris entre 2 et 8, et
p est compris entre 0 et 3.
